# EUROPEAN PATENT SPECIFICATION

(11) **EP 4 380 664 B1**
(45) Date of publication and mention of the grant of the patent: **02.04.2025**
(21) Application number: 22757330.0
(22) Date of filing: 04.08.2022
(51) Int. Cl.: A61M 25/02, A61M 5/14, F16L 3/00

(54) **CONDUIT SECUREMENT DEVICE**
LEITUNGSSICHERUNGSVORRICHTUNG
DISPOSITIF DE FIXATION DE CONDUIT

(30) Priority: 06.08.2021 GB 202111387
(43) Date of publication of application: 12.06.2024
(73) Proprietor: Javelo Health Limited, Edinburgh EH4 2HS (GB)
(72) Inventor: BARNETT-VANES, Ashton, Edinburgh EH4 2HS (GB); KEALY, Terence, Bray, A98 Y6W0 Co. Wicklow (IE)
(74) Representative: HGF
(86) International application number: PCT/GB2022/052057
(87) International publication number: WO 2023/012484

(56) References cited:
- WO-A1-2017/075218
- DE-A1- 102015 213 686
- US-A- 4 505 006
- US-A1- 2007 142 785
- US-A1- 2011 130 725

## Description

### Field of the Invention

The invention relates to the field of attachment of a conduit in particular for use in a clinical setting, in particular the attachment of catheters, intravascular lines and the like to a patient.

### Background to the Invention

Medical treatments commonly involve infusing fluids into or withdrawing fluids from a patient, by connecting a fluid line to a body cavity such as the thorax, abdomen, cranium or luminal conduit such as a vein, artery, spinal cord, urethra, bowel etc.

For example, an intravenous (IV) line may be used to deliver drugs, deliver or exchange plasma or to hydrate a patient, whereas bodily fluids may be removed via urinary or enteric catheterisation, or surgical drains may be placed within a cavity during or after surgery to drain contents and promote recovery.

A patient or body part can remain relatively mobile while a fluid line is attached and a patient may forget or be unaware of the fluid line, particularly for lengthy treatments or when regaining consciousness. A clinical setting may also be crowded with additional fluid lines and medical apparatus and clinicians may need to frequently work in close proximity to fluid lines, electrical conduits or the like. Accordingly, unplanned removal of lines from a patient is a common problem, can lower the quality of care and may lead to adverse clinical outcomes.

Unintended removal of fluid lines delivering even low cost drugs or fluids can lead to wastage, extend treatment time and cause distress to a patient. It is also known for dislodged lines to leave medical apparatus, such as a needle, in the patient which may require imaging or surgical intervention to remove. Furthermore, the consequence of unintended line removal in some settings can be severe or even fatal, particularly in emergency or surgical settings.

A number of approaches to secure fluid or other lines and isolate the entry point from forces applied to a line are commonly used.

Adhesive or tape can be used to secure a fluid line. Although this approach can be readily adapted to different types or diameters of line, hair, sweat or other fluids can weaken the adhesion to a patient's skin and at best only limited pull resistance can be achieved. These measures must be constantly renewed and, over time, adhesives and adhesive tapes can cause skin irritation. In some instances pain or injury may result if removed by pulling on a fluid line.

Devices are also known which mechanically retain a line within a retention device, to isolate the line from the entry point. In use of such devices, the attachment to the line is also at least to some degree independent of the attachment to a patient.

For example, the Statlock^{™} urinary catheter stabilisation device (manufactured by Bard Medical, Crawley, UK) includes an adhesive pad for applying to a patient's skin, to which is mounted a moulded retainer for a catheter. A lid latches shut over the catheter to retain it in place. The retainer is sized to retain a specific diameter of catheter, however, and offers limited resistance to longitudinal forces applied to a catheter secured in the device.

The Braidlock^{®} (manufactured by Braidlock Limited, Warrington, UK) instead employs a braided sheath to retain a catheter or IV line, which necks down under tension to resist longitudinal forces applied to the line. A Braidlock can accommodate a limited range of line diameters and installation, removal or exchange of IV lines is difficult due to the requirement for the line to be inserted or removed through the braided sheath.

WO2005051472 describes a medical securing device that similarly accommodates for only a limited range of line diameters, within open channels or grooves flanked by flexible "blades". The blades are disposed at an angle to the channel so as to, at least to some degree, resist pulling of a conduit or line in one direction. The device may be used as a conduit guide to assist in routing of conduits in a clinical setting, but additional stabilisation of a line would be required in the vicinity of the entry point. WO2017013114 describes a device with two opposed rows of resilient clamping elements within a housing and a guide channel extending therebetween. The clamping elements extend perpendicular to the channel, which limits the surface area that contacts a conduit. The perpendicular orientation of the clamping elements also further limits the amount of pull resistance that they are able to provide to a conduit in the channel.

Accordingly there remains a need to address one or more of the deficiencies encountered with existing fluid line securements and securement of conduits in general.

### Summary of the Invention

According to an aspect of the invention there is provided, according to claim 1, a conduit securement device, comprising:
a body defining a channel, for receiving a length of a conduit extending along the channel; and
a first array of resiliently deformable fins disposed along the channel extending from at least a first side and an opposite second side of the channel; and
a second array of resiliently deformable fins disposed along the channel extending from at least a first side and an opposite second side of the channel;
wherein each fin has an orientation that extends at an acute angle to a longitudinal direction along the channel;
wherein each of the fins of the first array has a first orientation with respect to the said longitudinal direction along the channel, and each of the fins of the second array has an opposite second orientation with respect to the said longitudinal direction along the channel; and
wherein the first array extends along a first portion of a length of the channel and the second array extends along a second portion of the length of the channel.

The body may further comprising a retention arrangement for resisting or preventing movement of a conduit laterally of out the channel.

In use, a conduit such as an IV line, catheter or the like is placed into the channel, and may be retained therein against being removed laterally from the channel by a retention arrangement. The device has a particularly compact configuration and so can advantageously be secured in a range of locations, while providing minimal interference with other apparatus, a patient's clothing or the like.

A conduit inserted in the channel typically slightly resiliently deforms at least the ends of the fins, and friction between the fins and the conduit provide resistance to longitudinal motion of the conduit along the channel, i.e. "pull resistance". In effect, the securement device isolates the conduit extending distally from the securement device from forces applied between the securement device and the conduit extending proximally from the device, and vice versa. The securement device thereby reduces the risk of disruption to the entry point of the conduit or associated medical apparatus to a patient.

The angled orientation of the array of fins ensures that when a conduit is inserted into the channel in use, the orientation of the fins is preserved and the fins thereby provide a greater "pull resistance" (resistance to longitudinal movement of the conduit in relation to the securement device) in one longitudinal direction than in the other. The fins are able to resiliently deform to accommodate a range of conduit diameters. The angled orientation of the fins further promotes a larger area of contact between a conduit and each of the resiliently deformed fins, further promoting pull resistance.

The pull resistance is provided by engagement of the conduit with multiple fins along a length of the conduit, thereby distributing forces along the conduit. In use with flexible or compressible conduits in particular this may limit the risk that a conduit might be "choked" (i.e. pinched or otherwise partially or completely occluded).

It will be understood that in a clinical setting, forces applied to a retained conduit may have a component in a longitudinal direction with respect to the conduit (i.e. axially) and a lateral component. The resiliently deformable fins are able to deform to accommodate such forces in a range of directions, while reducing the likelihood that the conduit will fold or pinch.

That is to say, the fins are at an angle to the longitudinal direction along the channel such that each fin defines an acute angle to one side and an obtuse angle to the other side of the fin, with respect to the longitudinal direction, wherein the acute angle is oriented towards one or other of a proximal or a distal end of the channel.

Each fin will have a free end adjacent to the channel and a fixed end further from the channel than the free end.

By the orientation of a fin, we refer to a direction from the free end to the fixed end of the fin, when the fin is in an undeformed configuration. A fin may be straight, between the fixed and free ends, such that the orientation between the fixed and free ends extends along a length of the fin.

It will be understood that when a conduit is inserted into the channel in use, the fins will resiliently deform to some degree, which may change the overall orientation of the fin, typically making the angle to the longitudinal direction of the channel more acute, and/or which may bend or otherwise deform the fin. The degree of deformation may also change when forces are applied between the conduit securement device and the conduit in use. For example a curvature may be introduced to at least a part of a fin, or the degree of curvature may be changed. A fin in use may furthermore be compressed or extended in use. A fin may be deformed to a different degree in different regions of a fin, for example to accommodate a cross sectional shape of a conduit inserted in the channel.

The fixed end of a said fin may be directly or indirectly coupled to the body. The fixed end may be generally fixed in relation to the body. By generally fixed we mean that the fixed ends may form part of a resiliently deformable structure, such as a moulding as disclosed below, and thus accommodate a small degree of deformation and thus movement of the fixed ends in use.

A said fin may be coupled (directly or indirectly) to the body along a portion or all of a length of a fin. It will be understood however that at least a part of the free end furthest from the body will remain uncoupled.

In some embodiments the or each fin is only coupled to the body at the fixed end. In use, such fins may be deflected and/or resiliently deformed in order to accommodate a conduit in the channel. A fin that is uncoupled along a length (or all of the length) of the fin from the free end is able to be deflected so as to accommodate a range of different conduit sizes.

By an "array of fins" we refer to multiple fins spaced apart, typically evenly, along at least a part, or all, of the length of the channel, with fins on opposed sides of the channel and having a common orientation with respect to a longitudinal direction along the channel. The orientation is defined with respect to the direction that the acute angle made by the fin in relation to the channel faces, with respect to the longitudinal direction. The angle at which the fins sharing a common orientation extend from the channel may vary along an array, or the angle of all of the fins in an array may be the same, at least in an undeformed state. An array can comprise any number of fins, or pairs of opposed fins, or opposed fins staggered along opposed sides of the channel. An array of fins may include the same, or a different number of fins on opposed sides of the channel. For example, along each side of the channel, an array of fins may comprise 2, 3, 4, 5, 6, 7, 8, 9, 10, 12, 15 or in some embodiments more fins, along the channel. The channel may comprise 2, 3, 4, 5, 6, 7, 8, 9, 10, 12, 15 or in some embodiments more fins along a side thereof, provided by one or more than one array. The length of the fins in an array of fins may vary along the channel and/or to opposed sides of the channel, for example to define a curved or convoluted channel as disclosed herein.

By proximal and distal, we refer to a direction or position with relation to a conduit, in use. A proximal end of a medical conduit typically extends from an apparatus or a container (such as a fluid container). A distal end of a medical conduit typically extends in use from an entry point to a patient's body cavity (such as a patient's vasculature), or to a contact point with a patient (e.g. a sensor positioned on or about a patient).

At least a part of the first and second arrays may extend along a shared portion of the length of the channel.

In an alternative embodiment, that is not in accordance with the claims, the first and second arrays may extend along the same portion of the length of the channel.

In an alternative embodiment, that is not in accordance with the claims, the first array of fins may extend to the first and second sides of the channel, and the second array may extend to a third and optionally fourth sides of the channel.

The first array of fins may extend around a first part (of the circumference or perimeter) of the channel and the second array of fins may extend around a second part of the channel. The first and second parts may together define the entire circumference or perimeter of the channel.

An array of fins may also extend from a third side and optionally a fourth side of the channel, wherein the third and fourth sides are orthogonal to the first and second sides.

An array of fins may also extend from the first, second, third side and optionally fourth side of the channel, along at least a portion of the length, and in some embodiments the entire length of the channel. The array of fins may extend substantially entirely around the channel, along at least a portion, or all, of the length of the channel.

The securement device may thereby be tolerant to a wider range of directions of forces applied to a retained conduit, in use.

In another aspect, that is not in accordance with the claims, the invention provides a conduit securement device, comprising:
a body defining a channel, for receiving a length of a conduit extending along the channel; and
an array of resiliently deformable fins disposed along the channel extending from at least a first side, an opposite second side of the channel; and a third side and an optionally an opposite fourth side of the channel; wherein the first and second sides are orthogonal to the third and fourth sides;
wherein each fin has an orientation that extends at an acute angle to a longitudinal direction along the channel; and wherein each of the fins has a common orientation with respect to the said longitudinal direction along the channel.

The array of fins may extend from the first, second, third and optionally fourth sides of the channel along at least a common portion of the length of the channel, or in some embodiments the entire length of the channel.

The device may comprise a first array of fins and a second array of fins. The first array of fins may extend along a first portion of the length of the channel. The second array of fins may extend along a second portion of the length of the channel.

The first and second arrays may extend along a common portion (or all) of the length of the channel. At least a part of the first and second arrays may extend along different portions of the channel.

Along at least a part of the length of the channel, the first and second arrays may together define the said array extending from the first, second, third and optionally fourth sides of the channel. That is to say, a given array may comprise a first and second sub-array. Along at least a part of the length of the channel, the first and second sub-arrays may be generally contiguous around the channel.

In some embodiments, the first array and second array of fins may, in use, close around a conduit.

In some embodiments, the retention arrangement comprises at least a part of an array of fins. For example, the second array may form part of a lid or closure and be operable to close around the conduit when the lid/closure is closed.

In some embodiments, two arrays of fins may come together in a "clam shell" or a mitred arrangement.

In an alternative embodiment, that is not in accordance with the claims, the first array of fins may have the same orientation as, the second array of fins.

In some embodiments, the channel is straight.

In some embodiments the channel is curved or convoluted, along at least a part of the length of the channel. The channel may comprise a curved or convoluted void pathway, as disclosed herein.

A flexible conduit, such as an IV line, can be placed in a curved or convoluted channel.

In use the curvature of the channel may supplement the pull resistance of the device. Tension applied to the conduit longitudinally (i.e. wherein at least a component of force is applied along a length of the conduit), acts to straighten the pathway of the conduit along the channel and this acts to urge the conduit against and further resiliently deform some of the fins, thereby increasing static friction between the fins and the conduit.

The channel may have a single curve. The channel may have two, or more directions of curvature. In some embodiments, for example, the channel is generally s-shaped or may comprise one or more undulations. In some embodiments having a channel with two or more directions of curvature, the channel extends along a plane. That is to say, the two or more curves are aligned. In some embodiments having a channel with two or more directions of curvature, comprises two or more than two orientations of curvature and does not extend along a plane. For example, a first curve along the channel may extend in a first plane and a second curve along the channel may extend along a second plane, wherein the second plane is at an angle (e.g. orthogonal to) the first plane.

By curved, such as curved channel or void pathway, we refer to a channel or pathway including at least one bend. A curved channel or pathway may include one or more than one radius of curvature. By convoluted, we refer to a channel or pathway including at least two bends in generally opposite but not necessarily co-planar directions along a length thereof. A convoluted channel or pathway can be generally serpentine or s-shaped, for example. The curvature of the channel may be defined by the free ends of the fins (for example, the length of the fins may vary along the channel). Alternatively, or in addition, the curvature of the channel may be defined by one or more ribs, as disclosed hereinbelow.

In some embodiments, the body comprises an inner portion and a support portion disposed laterally outside of the inner portion along at least the first and second sides of the channel, wherein the inner portion defines the channel and comprises the array of resiliently deformable fins. The inner portion may comprise one or more fin components, which may be removable, movable or reconfigurable, as disclosed herein.

In some embodiments, the support portion comprises a material having a first stiffness and at least the fins of the inner portion comprise a material having a lower second stiffness.

In accordance with another aspect, that is not in accordance with the claims, a conduit securement device comprises:
a body comprising an inner portion and a support portion;
   wherein the inner portion defines a channel, for receiving a length of a conduit extending along the channel; and
   wherein the inner portion comprises an array of resiliently deformable fins disposed along the channel extending from at least a first side and an opposite second side of the channel; wherein each fin has an orientation that extends at an acute angle to a longitudinal direction along the channel, and wherein each of the fins has a common orientation with respect to the said longitudinal direction along the channel;
and wherein the support portion is disposed laterally outside of the inner portion along at least a first and an opposite second side of the channel; and the support portion comprises a material having a first stiffness and at least the fins of the inner portion comprise a material having a lower second stiffness

The first and second materials may thus be selected to optimise the performance of the securement device. A lower stiffness (Young's modulus) resilient material may be used for the fins (or the entire inner portion), to facilitate resilient deformation and allow the fins to better conform to the shape and contours of a conduit in use. Whereas a higher stiffness material may be used by the support portion, to resist such resilient deformation from being transferred outwardly.

The entirety of the support portion and/or the entirety of the inner portion may be formed from the respective first or second materials.

The first material may be a different material to the second material or may be of the same material or class of material, but subject to different processing so as to vary, for example, material density or morphology.

The inner portion may be coupled to, for example between or within, the support portion. The inner portion and support portion may be formed separately and coupled together during manufacture.

The inner portion and support portion may be removably coupled to one another, for example to allow for use or selection of an inner portion having a suitably sized or configured channel void pathway and/or resilience of the fins thereof for a particular purpose, with a single configuration of support.

In some embodiments, the inner portion is moulded within the support portion. For example the support portion and inner portion may be co-moulded together.

The support portion may comprise or define a recess, and the inner portion may be located in the recess. The support portion may define proximal and/or distal ends of the channel. For example, the support portion may define a recess (e.g. a box or enclosure for the inner portion) and the channel may extend through openings or indents in the walls of the recess.

As disclosed herein, at least the fins of the inner portion comprise or consist of an elastomeric or otherwise resiliently deformable material.

The support portion, or at least a part thereof, may be rigid or semi-rigid. By semi rigid we mean that the support portion may be flexible to some degree, but have sufficient rigidity to resist the forces applied via the fins when deformed by a conduit in the channel. The support portion may for example comprise or be formed from a moulded or machined plastics material, such as polypropylene, acrylonitrile butadiene styrene (ABS) or a polycarbonate.

In an alternative embodiment, opposed fins may be longitudinally offset from one another on alternating sides of the channel.

The securement device of any of the aspects or embodiments disclosed herein may be adapted to limit the inversion or prolapse of the fins, in use when a longitudinal force is applied between a conduit in the channel and the securement device in the same orientation as the fins. Such adaptation may further improve pull resistance.

The ends of the fins in an array of fins that contact a conduit in use may be joined together, along one or both sides of the array.

The securement device may comprise one or more barriers or ribs extending along at least a part of the channel, between ends of the fins extending from the channel to opposed sides of the channel. In particular in embodiments wherein the fins of an array of fins have a free end, a said rib or barrier extends partly between the free ends.

A said rib or barrier may be rigid or semi-rigid, along at least a part of a length of the channel.

A said rib may be resiliently deformable along at least a part of the length of the channel, such as at least at the proximal and/or distal end of the channel. A resiliently deformable rib may reduce the risk of bending or crimping of a conduit. An array (or sub array) of fins may also extend partly between the free ends of another array of fins to perform a similar function.

A said rib or barrier may at least partly define a void pathway.

The device may comprise more than one rib or barrier. For example, an array of fins may extend from a first and opposed second side of the channel, and a rib may extend from a third and optionally a fourth side of the channel, between the fins.

A said rib or barrier may define a curved or convoluted channel.

A rib or barrier may comprise part of the body, or a said support portion and/or lid, for example.

Some embodiments may comprise one or more reconfigurable ribs or barriers. A rib or barrier may for example be operatively coupled to a button or slider, or a moveable wall portion as disclosed herein, whereby the extent to which the rib or barrier extends between the fins of an array can be reconfigured to provide for some adjustment of the pull resistance of the device.

The securement device can comprise more than one fin component making up the array or arrays and defining the or each channel. It will be understood that the arrays and channels of the securement devices disclosed herein may be constructed using a variety of fin components. For example, an array of fins can comprise multiple parts, for example each part defining the fins on one side of the channel, or each part defining fins extending partially around the channel. An array of fins can, in some embodiments, be formed as a single unit for example by moulding. Conversely, a given fin component can define at least a part of more than one array of fins.

In relation to any of the aspects of embodiments disclosed herein, an array of fins may have a range of configurations as follows.

When the fins are in an undeformed state (when no conduit is present in the channel), the channel may define a void pathway. That is to say, in a lateral direction with respect to the channel, the channel may comprise a gap between opposed fins (e.g. between the fins to the first and second sides of the channel).

The width (laterally) of the void pathway may define a minimum diameter of conduit that may be secured within the securement device. The minimum diameter may for example be at least 105%, 110%, 120% or 130% of the width of the void pathway.

In cross section through the channel (i.e. viewed longitudinally along the channel), the void pathway may be square or rectangular, when the fins are undeformed. That is to say, the ends of the opposed fins define two, three or four sides of a square, rectangular or diamond shaped in cross section void pathway.

The void pathway may be circular, oval or polygonal in cross section. That is to say ends of the array of fins facing the channel may define a part of (typically more than half of) a circle, oval or polygon.

Other cross sectional shapes of the void pathway are also possible. For example, the ends of the opposed fins may be provided with a curvature or a lip, to assist, at least in part in engaging with, cupping around and/or retaining a conduit in the channel in use.

Each fin of the array of fins may have a fixed end, that is generally fixed in relation to the body (other than minor movements which, in some embodiments, occur during resilient deformation of a fin component in use), and a free end, moveable in relation to the body to accommodate a conduit inserted into the channel.

The angle may be defined between the fixed and free ends. In an undeformed state, the angle of a said fin may for example be between around 10 and 85 degrees, 20 and 80 degrees, or between around 30 and 75 degrees, or between around 45 and 70 degrees. The angle may be around 5, 10, 15, 30, 45, 50, 55, 60, 65, 70, or 80 degrees. In an undeformed state, the angle of each fin of an array of fins with respect to a longitudinal direction of the channel may be substantially the same.

In some embodiments, the angle of the fins of an array of fins may vary along the channel. For example where the channel is curved or convoluted, adjacent fins may be substantially parallel to one another, such that the angle thereof with respect to a curved or convoluted channel may change along the length of the channel.

It will be understood that the angle between the orientation of the fins and channel when the fins in a deformed state, when a conduit is inserted into the channel, may change and that the amount by which the angle changes may depend on the diameter and flexibility of the conduit and indeed become non-uniform along the channel.

In an undeformed state, the fins of an array of fins may be substantially straight, between their fixed and free ends. In some embodiments, in an undeformed state, the fins of an array of fins are curved or J-shaped between their fixed and free ends, or otherwise non-straight. Such configurations may, in some embodiments, provide for an increased surface area of contact between the fins and a conduit, in use.

The fins may be composed of any suitably resilient material, including for example resilient plastics materials or elastomeric materials, such as silicones, closed cell neoprenes, polyurethanes, polystyrenes, polyolefins and other such materials known by the skilled person for use in medical applications. The fins may comprise a resilient component and a coating component. For example, a coating or surface treatment (e.g. a roughened surface, or alternatively a smoothed surface, or a "tacky" coating such as a polyurethane coating) may be selected to more effectively frictionally engage with a particular class of conduit, such as IV lines.

A fin may comprise more than one resilient material. For example, a portion of a fin (such as a lip or other formation that in use cups around or over a conduit to retain the conduit within the channel against being removed laterally from the channel) may comprise or be formed from more resilient (i.e. stiffer) material, to aid retention of the conduit within the channel.

The resilience of a fin may vary along its length for example to increase from the free to fixed end thereof.

The materials may be selected to have suitable solvent or temperature resistance, for cleaning, as known in the art.

The resilience of the fins may be selected for a particular purpose, based upon typical pull forces that could be expected to be exerted between the conduit and the securing device in normal use. A required load might for example be related to the weight of equipment or apparatus from which a conduit extends. For example, for securement of IV lines, it is desirable for the securement device to be able to isolate the IV line entry point to the vein from at least dropping a fluid bag. Such bags (e.g. of saline, plasma, etc) typically contain around 1 litre of aqueous fluid. A securement device might accordingly be required to isolate a conduit (e.g. drain) to the distal side of the device from longitudinal forces applied to the conduit to the proximal side of the device of around 1 to 10 N, or 1 to 5 N, or around 1 or 1.5 to 3 N is applied between the device and a retained IV line.

In some embodiments a greater load resistance can be anticipated, such as with use of heavier apparatus such as a surgical drainage device, weighing around or greater than about 10, 20, 30, 40, 50, 60 or 80 kg or sometimes more. A securement device might accordingly be required to isolate a conduit (e.g. drain) to the distal side of the device from longitudinal forces applied to the conduit to the proximal side of the device of around 10 to 200 N, or 50 to 150 N or 70 to 100 N, or around or greater than about 10, 20, 30, 40, 50, 60 or 80 N or sometimes more.

It will be understood that the securement device may isolate the longitudinal forces but permit some degree of movement of the conduit in the channel. A tolerance of such movement may for example be around or less than 20 mm, 15 mm or 10 mm, under the action of a given longitudinal force.

The retention arrangement functions to retain a conduit within the channel, against lateral movement out of the channel.

The retention arrangement may comprise a narrowed entry to the channel, defined by the body. The narrowed entry may be provided with a width less than the width of the conduits with which the securement device is sized for use, whereby, in use the body is flexed to temporarily widen the opening and/or a conduit is squeezed to fit through the channel. The narrowed entry may be less than a width of the channel void pathway.

The retention arrangement may comprise a closure across or over at least a part of the channel. For example, the retention arrangement may comprise one of more straps, which can be releasably secured across the channel.

In some embodiments, the retention arrangement comprises a lid or cover, for attaching over at least a part of the length of the channel. A lid may be hinged in relation to the body, for example by an active hinge, and secured by way of a latch, clip or any other suitable fixing. A cover may be removably attached over at least a part of the length of the channel. The cover may be attached by way of any suitable fixing, such as a clip, latch or the like.

The retention arrangement may be coupled to the support portion.

The support portion may comprise the retention arrangement. For example, in embodiments having fins extending to a third and optionally a fourth side of the channel, or around the channel, the retention arrangement may comprise a lid or a closure over the channel, wherein the lid or closure in some embodiments comprises a fin component or components.

The lid or closure forming part of the support portion may comprise an insert portion, for example an insert portion comprising an array of said fins which in use (when the lid or closure is positioned over the channel) extend from a third side of the channel.

In embodiments wherein the support portion comprises a material having a first stiffness and at least the fins of the insert portion comprise a material having a lower second stiffness, the lid or cover may comprise the material having the first stiffness.

The securement device may in some embodiments be reconfigurable.

A reconfigurable securement device may be reconfigurable, between at least a first configuration and a second configuration, to change an angle of at least some of the fins in relation to the channel, change a width of the void pathway along at least a part of a length of the channel, change a curvature of the channel, or change the orientation of at least some of the fins, or the like.

A said support portion and/or a said inner portion may be reconfigurable.

Reconfiguring may adjust the pull resistance applied by the device to a conduit retained in the channel, or may adjust a direction of the pull resistance applied by the device to a conduit retained in the channel, or may adjust the size of the channel (and thus the diameter of conduit which may be retained therein).

The body (for example a support portion thereof) may comprise a button or slider for changing the configuration of the securement device. A button or slider may be operable to move the device between the first configuration and the second configuration.

In use, a conduit may be loaded into the channel when the device is in the first configuration, and then the device moved to the second configuration.

The curvature of at least a part of the channel may be changed by moving the device between the first and the second configuration.

In the first configuration, the channel may be straight. In the second configuration, the channel may be curved. The curvature of at least a part of the channel may be increased, by moving from the first to the second configuration.

The width of channel void pathway along at least a part of the length of the channel may be reduced, by moving from the first to the second configuration.

Movement between the first and second configurations may be achieved by pressing a button, or moving a slider (e.g. a ramped slider) so as to move all or a portion of the fins laterally in relation to the channel. All or a portion of the fins to the first and second side of the channel may be deflected laterally, to impart a curvature to , or change a curvature of, the channel.

An arrangement for deflecting the fins laterally may be conveniently provided within a support portion as disclosed herein. A button or slider may deflect a movable inner wall of the support portion laterally and/or longitudinally, or may prevent or restrict lateral motion of the support portion.

A slider may be laterally or longitudinally movable and coupled to or engaged with at least one of the fins. For example at least some of the fins may engage or be coupled to a said moveable portion, along the at least a part of the length of the channel along the first and/or second side of the channel. A said moveable wall portion, button or slider may be coupled to or engaged with one or more fin components.

Movement of the slider may, in use, reconfigure the angle of the associated fins. For example a conduit may be retained in the channel when the securement device is in a first configuration and the slider operable to move the device to a second configuration. The ends of the fins engaged with the conduit will remain substantially in the same position in both configurations, and the ends of the fins coupled to the slider will move longitudinally, thereby changing their angle. The fins coupled to the slider may be moved between a first configuration in which they are generally perpendicular to the channel, to a second configuration in which the fins are at an angle to the channel, as disclosed herein.

The angle of the fins may be changed to change the orientation of the associated fins.

A button or slider may be indexed, and be provided with at least two indexed positions defined by the first and second configurations.

The securement device may be reconfigured by removing and replacing one or more fin components. For example, in some embodiments, each of one or more arrays may comprise or consist of a removably coupled fin component, whereby the device can be reconfigured by removing the fin component and recoupling the fin component with the fins thereof in an opposite orientation. This may facilitate, for example, use of fin components of different size, resilience, side of void pathway, curvature etc. within a given body. Where placement of a device on a patient or equipment is sensitive, this may reduce the likelihood of error and/or improve efficiency in a clinical setting.

The securement device may comprise one or more rotatable fin components. One or more fin components may be rotatably coupled to the body or support portion. The securement device may be reconfigured by rotating one or more fin components to reverse the orientation of the fins thereof.

Changing the orientation of a fin component allows for the selection of the orientation of an array of fins, and thus of greatest pull resistance provided by the array.

In some embodiments, a device may comprise two or more arrays, at least one of which is reconfigurable to change the orientation of the fins thereof, whereby the securement device can be selectively capable of providing a substantially equal pull resistance in both longitudinal directions (with respect to the channel), or to provide a greater pull resistance in a selected longitudinal direction.

The securement may comprise a first channel and a second channel. Some embodiments may comprise more than two channels.

The securement device may comprise more than one body, each defining a channel.

The securement device may comprise a body defining more than one channel.

The first and second channels may extend generally parallel to one another.

The first and second channels may share a common first end and/or a common second end.

In some embodiments, that are not in accordance with the claims, the first channel comprises a first array of fins and the second channel comprises a second array of fins. The first and second arrays may be in different orientations., such that the directionality of the securing device may be selected.

In some embodiments, that are not in accordance with the claims, the first channel comprises a first array of fins, and the second channel comprises two second arrays of fins in opposite orientations with respect to a longitudinal direction of the second channel. A conduit may be inserted so as to selectively provide a single direction of pull resistance, or pull resistance in both directions.

Two or more channels may extend end to end. In some such embodiments, the securement device comprises a branched channel arrangement. That is to say, the device comprises a main channel that extends at an end thereof to a said first and second channel. Each of the main, first and second channels may comprise an array of fins, or more than one array of fins, in any combination or sequence of orientations.

Indeed the device can comprise two pairs of first and second channels, each pair sharing common first and/or second ends, thereby providing a range of pathways for a conduit.

Thus the degree of pull resistance provided by the device to a conduit secured therein in each direction can be selected with greater resolution.

In addition to, or alternatively, securing devices having multiple channels may comprise channels having more than one width of void pathway, or may comprise arrays of fins having more than one resilience. Such embodiments may provide, in use, for a particular pathway to be selected to accommodate a particular diameter or flexibility of conduit.

The securing device may further comprise an external attachment, for attaching the securing device to a patient or structure, wherein the external attachment is coupled to the body.

The external attachment may for example comprise a band or strap for attachment around a patient's limb. The external attachment may comprise a garment, or garment portion, such as a vest, undergarment or limb covering. The external attachment may comprise an adhesive portion, for example in the form of a tape or an adhesive patch or strip, which can be applied to a patient's skin or clothing, or to a structure such as a piece of furniture or a trolley. The external attachment may comprise a releasable fastening, such as a clip or a component of a hook and loop fastening.

An external attachment for attaching the device to a patient may comprise an adjustable band or strap. Adjustability may be provided by any suitable means, such as hook and loop fastenings, buckles, ratchets, laces or the like.

The body may be attached to (optionally releasably) or at least partially embedded in or surrounded by the external attachment. For example, the external attachment may comprise a plastic or elastomeric material and the body, or at least a support portion thereof may be a stiffer material such as a polypropylene material, at least partially embedded in or surrounded by the plastics/elastomeric material of the external attachment.

The body may be connected to the external attachment, for example via one or more clips, fixings or loops or the like.

The body may be removable from the external attachment, for example for cleaning. The body and external attachment may be integral, for example formed from a single material.

The material selection for the securement device or component parts thereof may be determined according to the clinical setting. It will be understood by the skilled person that in some circumstances, such as for surgical or emergency applications, the device may be adapted for single use. Whereas for other applications, for example where use of the device may be required over a longer period, material selection for patient comfort, cleaning of the device and/or sterilisation and re-usability of the device may be desirable.

In some embodiments the securement device or one or more component parts thereof is conveniently made from a wipe-clean material. A wipe clean material is a material having a substantially non-porous surface. Examples include plastics materials or elastomeric materials, such as silicones, closed cell neoprenes, polyurethanes, polystyrenes, polyolefins and other such materials known by the skilled person for use in medical applications. The securement device may comprise cleanable or sterilizable fabrics, such as plastics or elastomer coated fabrics.

In some embodiments, the securement device, or on or more component parts thereof, is made from a recyclable material, such as a thermoplastics material, or a biodegradable material, such as a biodegradable plastics material. Biodegradable plastics materials, such as a cellulosic or other plastics materials based upon plant-based polymers or resins are known in the art.

One or more components of the securement device may be moulded (that is to say formed by moulding, such as compression or injection moulding or the like). For example fin components, one or more parts of the body, the external attachments etc. may be moulded.

One or more components of the securement device may be overmolded or comoulded with one or more further components. For example the support portion and inner portions may be comoulded, the external attachment may be overmoulded around at least a part of the body.

The securement device or component parts thereof may be provided with an antipathogenic (such as antibacterial, antifungal or antimicrobial) coating, or may be impregnated with an antipathogenic component. Many such antipathogenic components are known in the art, including metal colloidal compositions, such as silver or copper based, quaternary ammonium compositions or polymers, biguanide polymers and many more.

The securement device or component parts thereof may be provided with a luminescent coating or may be impregnated with a luminescent pigment or dye. This may assist for example in locating the securement device at night or in low light conditions, or may serve as a reminder to a patient that they are still wearing the securement device.

The invention extends in another aspect to a medical system comprising a medical conduit secured to a securement device according to aspects and embodiments disclosed herein, wherein the conduit extends along a length of a channel of the securement device.

The conduit may be flexible, semi-flexible or rigid.

The conduit may be a fluid line, such as an IV line, a venous line, an arterial line, a drain or the like. The conduit may be a gas line, for example to deliver anaesthetic or oxygen. The, or each, conduit may be an electrical or optical conduit, such as might extend from monitoring devices applied externally or transcutaneously to a patient (e.g. compartment monitors, electrical or optical sensors and the like).

The conduit may be flexible and urged into a curved or convoluted pathway by the array of fins.

The securement device of the medical system may further comprise an external attachment.

The external device may be attached or attachable to a patient or structure, for example around a patient's limp, head, neck or torso, or to or around a trolley, bed frame or the like. The securement deice may be attached or attachable to a stand, such as a drip stand, ICP monitoring stand or the like.

The medical system may comprise two or more conduits (of the same or different types or diameters) secured to the securement device and extending along respective channels thereof.

Further alternative or optional features of the securement device correspond to those of other aspects or embodiments of the securement device disclosed herein.

In another aspect there is provided a method of securing a conduit comprising:
providing a securing device in accordance with the aspects and embodiments disclosed herein; and
inserting a conduit laterally into a channel thereof.

The method may comprise operating a retention arrangement. A retention arrangement can be operated for example by securing a strap over the channel, closing a lid or the like.

The method may comprise reconfiguring securing device between at least a first configuration and a second configuration, to change an angle of at least some of the fins in relation to the channel, change a width of the void pathway along at least a part of a length of the channel, change a curvature of the channel, or change the orientation of at least some of the fins, or the like.

The securing device can be reconfigured before inserting the conduit into the channel, or after inserting the conduit in the channel. The securing device can be reconfigured before, or after operating a retention arrangement.

The method may comprise urging a flexible conduit into the curved or convoluted pathway.

The method may comprise attaching the external attachment to a patient or structure.

The external attachment may comprise a band or garment, and the method may comprise wrapping the band around a patient's limb or other body part, or placing a patient's limb or body part through or into the external attachment.

The external attachment may comprise an adhesive tape, and the method may comprise placing the securement device on a patient or a garment, and applying the adhesive tape over a part of the securement device. The external attachment may comprise an adhesive surface and the method may comprise preparing or exposing the adhesive surface and applying it to a patient.

The external attachment may comprise a clip or a clamp, and the method may comprise attaching said clip or clamp to a patient's clothing, bed frame or the like.

The method may comprise attaching the external attachment before and/or after securing a conduit to the securement device.

It will be understood that the method may comprise securing two or more conduits to the securement device. Two or more conduits may be secured at the same time. In some embodiments, one or more conduits may be attached at a different time to one or more other conduits. For example a clinician may in some circumstances return to a patient and secure and/or remove conduits - e.g. to replace an IV drip.

The method may comprise removing a conduit from the securement device. Removal may be effected by reversing the securement methods disclosed herein. Removal may be effected by breaking one or more said straps, for example by cutting one or more straps to release a conduit or conduits.

In another aspect, the invention relates to the use of the securement device disclosed herein to secure a conduit, such as a medical conduit. The use may be to secure a medical conduit with respect to a patient, for example proximate to an entry point, such as a cannula entry point.

It will be understood that further and optional features of each aspect of the invention correspond to further and optional features of any other aspect of the invention. The methods disclosed herein may for example comprise use of any of the features of the securement device or medical system disclosed herein; whereas the securement device and medical system may include any apparatus required for performance of the method.

By "lateral" and "longitudinal" and related terms, we refer to orientation or direction with respect to the channel, a void pathway therethrough, or a conduit as the case may be. For example with respect to a conduit a longitudinal direction is generally along an axis of the conduit or along a length of a curved conduit. The channel may extend from a first end to a second end of the body, and the longitudinal direction with respect to the channel may be generally in the direction between the first and second ends. A longitudinal direction may be defined along any part of a curved or convoluted channel. Similarly, a "lateral" direction may be defined at an angle to a longitudinal direction. For example a lateral direction may be generally radial with respect to a conduit axis, or generally perpendicular to a longitudinal direction of a channel. Where we refer to a longitudinal or "pull" force applied to the conduit, we refer to a force applied to the conduit in relation to the securement device (i.e. as might occur through movement of either), at least a component of which is parallel to the conduit.

The terms comprise or comprises, herein refer to an apparatus or method including the recited features and optionally further features. The terms comprise or comprises also encompass an apparatus or a method that consists only of the recited features.

While the invention has been described principally in relation to securement devices, systems and methods for medical applications, a securement device may also be provided for use with other types of conduits and in other settings where routing, securement and isolation of lengths of cables from tension is desired, such as electrical conduits, for ropes, braided cables etc.. References herein to medical conduits and so forth should therefore not be considered to be limiting.

### Description of the Drawings

Example embodiments will now be described with reference to the following figures in which:
Figure 1(a) and (b) show perspective views of examples of a conduit securement device;
Figure 1(c) shows a plan view of the device of Figure 1(b) with a conduit secured in the channel;
Figure 2 shows a plan view of another example of a conduit securement device, with a medical conduit positioned in the channel;
Figures 3(a) and 3(b) show perspective views of the device of Figure 2 in each of two configurations;
Figures 4(a) and 4(b) show perspective views of a further example of a reconfigurable securement device, in each of two configurations;
Figure 5(a) shows a perspective view of a conduit securement device with a convoluted channel and Figures 5(b) and 5(c) show the securement device with flexible conduits positioned along the convoluted channel;
Figures 6(a) and 6(b) show plan and cross sectional end views of a conduit securement device with a lid and arrays of fins around the channel;
Figures 7 and 8 show securement devices with two channels;
Figure 9 shows a securement device with a branched channel arrangement;
Figures 10, 11 and 12 show reconfigurable securement devices;
Figures 13(a) to (c) show images of a securement device with a body defining ribs between an array of fins; and
Figure 14(a) shows a perspective view of a securement device having a convoluted channel with multiple directions of curvature, and Figure 14(b) and (c) show exploded perspective views of insert portions thereof.

### Detailed Description of Example Embodiments

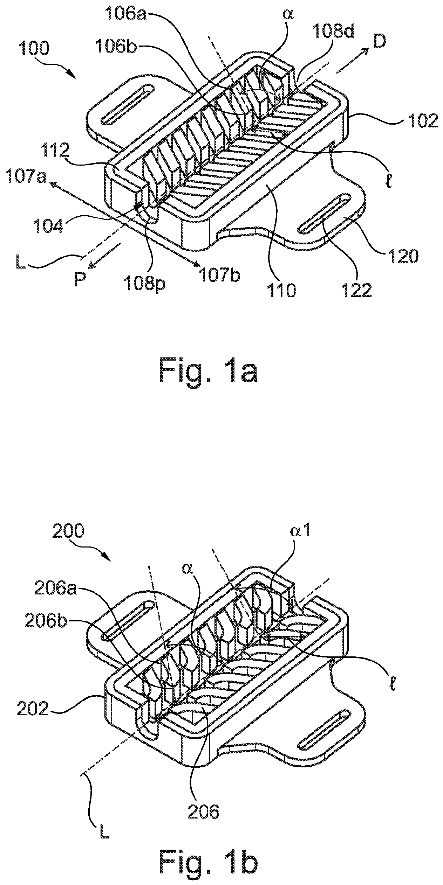

Figure 1(a) shows a securement device 100 for securing a conduit, in particular a medical conduit. The device 100 has a body 102 defining a channel 104. The channel includes an array of resiliently deformable fins 106, extending from a first side 107a and a second side 107b of the channel. The device 100 is shown with the fins in an undeformed configuration.

The fins each have an orientation that extends at an acute angle α with respect to a longitudinal direction L along the channel. The angle α is measured generally along a length / of a said fin 106, taken from a fixed end 106a to a free end 106b thereof. Each of the fins 106 has a common orientation. That is to say, the acute angle α of each fin, between a line defining the orientation of the fins extending from the free end 106b and the fixed end 106a of each fin and the longitudinal direction L of the channel, faces generally in the same way in relation to the longitudinal direction L (i.e. towards the same proximal 108p or distal end 108d of the channel).

In use, a conduit such as an IV line, catheter or the like is placed into the channel. The size of the device 100, and in particular the width of the channel 104 can be selected such that the conduit resiliently deforms at least the ends of the fins, whereby friction between the fins and the conduit provide resistance to longitudinal motion of the conduit along the channel, i.e. "pull resistance".

In use, the securement device isolates the conduit extending distally from the securement device from forces applied between the securement device and the conduit extending proximally from the device, and vice versa. The securement device thereby reduces the risk of disruption to the entry point of the conduit or associated medical apparatus to a patient.

The angled fins provide a greater "pull resistance" distally (in the direction D). As a conduit is pulled in the direction D, the ends of the fins 106 are to some degree dragged by friction into compression, or further compression, thereby increasing the engagement with the conduit. When pulled in the direction P, although there will be a degree of friction (and thus pull resistance) between a conduit and the fins, the friction therebetween will tend to elongate the fins and sharpen the acute angle α, reducing the deformation of the fins.

The fins are able to deflect and resiliently deform to accommodate a range of conduit diameters.

It will be understood that in a clinical setting, forces applied to a retained conduit may have a component in a longitudinal direction with respect to the conduit (i.e. axially) and a lateral component. The resiliently deformable fins are able to deform to accommodate such forces in a range of directions, while reducing the likelihood that the conduit will fold or pinch.

The resilience or other properties of the fins (such as surface texture, or material), the length and/or width of the channel can be selected according to a particular purpose.

The fins 106 in the array are parallel to one another (when undeformed) and are each at the same angle to the longitudinal direction of the channel. In alternative embodiments disclosed herein, the angle in relation to the channel can vary along the channel.

In the embodiment shown, the body 102 includes a support portion 110 and an inner portion 112. The support portion is laterally outside of the inner portion and, in the example shown, the support portion defines a recess with indents 110a at the ends of the channel 104. As discussed in further detail below, the inner portion may in some embodiments be removable. In other embodiments, an inner portion may not be removable and may for example by bonded or moulded within the support portion.

The support portion 110 is moulded from a relatively stiff and inflexible plastics material, such as polypropylene. The inner portion 112, which includes the array of fins 106, is moulded from a resiliently deformable material, such as medical grade silicone, having a lower stiffness. In the embodiment shown, the entire inner portion (including the fins) is moulded from a single material, however, in alternative embodiments more than one material may be used, wherein at least the fins 106 have a lower stiffness than the outer support portion 110.

The inner portion 112 is removable from the support portion 110, to allow for the device 100 to be reconfigured with different inner portions 112.

Provision of the inner and support portions having different stiffnesses allows for their stiffness to be optimised. For example, the fins may be made to have a comparatively low stiffness, so that they are better able to conform to and grip a conduit, with the support portion providing overall resistance against widening of the channel.

The securement device 100 also include a base 120 with slots 122, which function as an external attachment, for attaching the securing device to a patient or structure using a band or strap (not shown) threaded through the slots. The base 120 is co-moulded, in the embodiment shown, with the body 102.

Optionally, the strap may be routed over the open upper face of the channel 104, to act as a retention arrangement over the channel to prevent a conduit from being pulled laterally out of the channel. In alternative embodiments, the device 100 alternatively includes a lid or flap (not shown) operable to close over the channel and function as a retention arrangement.

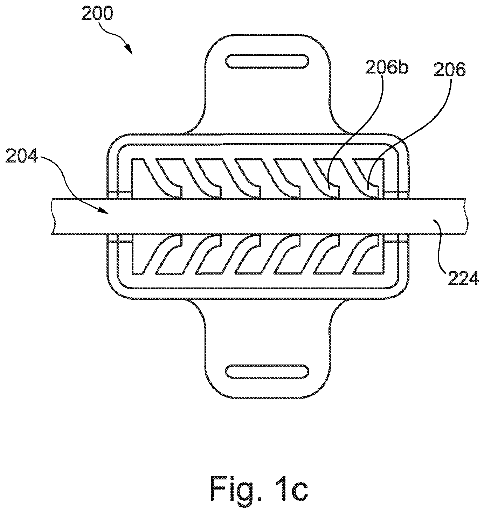

Figure 1(b) shows another securement device 200. Features in common with the securement device 100 are provided with like reference numerals incremented by 100. Unlike the fins 106 of the device 100 (which are straight in the direction from their fixed to free ends 106a, 106b, when the fins are in an undeformed configuration as shown in the figure), the fins 206 of the device 200 are "J-shaped", that is to say provided with a curvature towards their free ends 206b. The free ends 206b of the fins 206 are thus at a more acute angle to the longitudinal direction of the channel L, than the angle α1 of the fixed ends 206a. The fins' orientation (the angle α defined between the line extending between the fixed and free ends, and the channel orientation L) is also less (i.e. more acute) than the angle α1. In the embodiment shown, the free ends 206b are approximately aligned with the longitudinal direction of the channel L. In use, the J-shaped fins 206 may provide a larger surface area of contact with a conduit. This is illustrated in Figure 1(c), which shows the device 200 with a conduit positioned in the channel 204. The fins 206 are resiliently deformed against the conduit 224, with an area of the face of each of the fins 206 near their free ends 206b in contact with the conduit 224.

Other non-straight configurations are possible, such as a gradual curvature, or a kink midway along each of the fins.

The fins of the devices 100, 200 are shown in their undeformed configurations. As shown in the figures, the free ends 106b, 206b of opposed fins (to the first and second sides of the channel do not meet, such that the channel defines a void pathway between the free ends of the opposed fins.

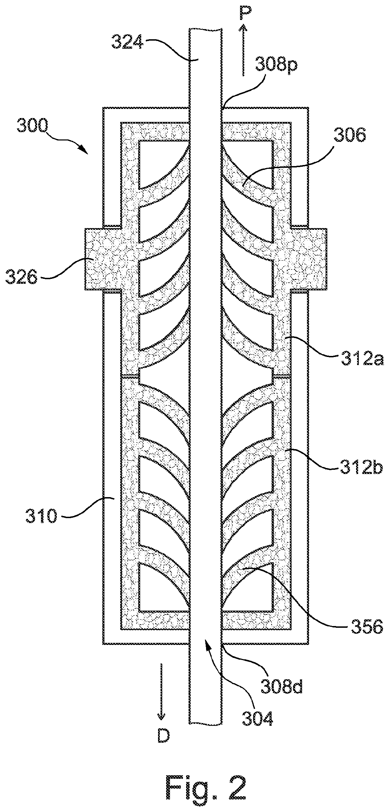

Figure 2 shows another securement device 300. Features in common with the device 100 are provided with like reference numerals incremented by 200.

The securement device 300 has a first array of fins 306 extending along a first portion of the channel 304, and a second array of fins 356 extending along a second portion of the channel 304. Each of the fins of the arrays 306, 356 are resiliently deformable, and extend at an acute angle α to the longitudinal direction of the channel 304.

A length of a conduit 324 is shown positioned in the channel, and the fins 306, 356 are resiliently deformed to accommodate and retain the conduit 324 in the channel 304.

The first array of fins 306 have a first orientation, and are oriented in the direction P towards the proximal end 308p of the channel. The second array of fins 356 have an opposite second orientation, and are oriented in the direction D towards the distal end of the channel. Thus, the device 300 provides pull resistance to the conduit in both directions, by virtue of additional compression of one or other of the first or the second array 306, 356 of fins when tension is applied longitudinally to the conduit.

It will be understood that the device 300 can be used in either orientation, and the terms proximal and distal are used for explanatory purposes only. That is to say, in use the end 308d of the channel may be oriented proximally, for example.

The device 300 can optionally comprise the further features of the body as discussed above in relation to the device 100.

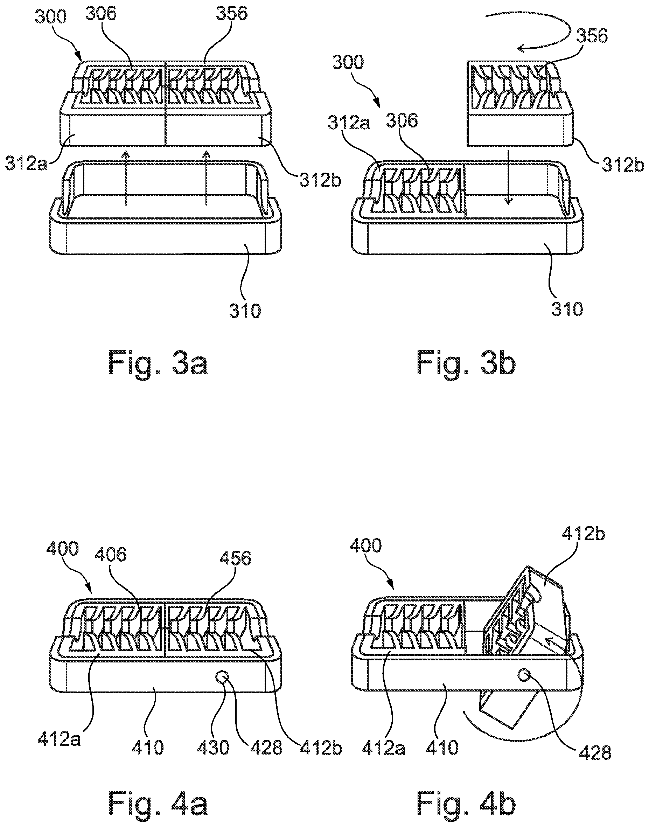

In one embodiment, the device 300 is reconfigurable and comprises first and second inner portions 312a and 312b that are removable. In use, one of the inner portions 312a, 312b can be removed and re-inserted into the support portion 310 with the orientations of the fins reversed, such that the device 300 is reconfigured to have two arrays of fins 306, 356 oriented in the same direction. Figures 3(a) and 3(b) illustrate how the device 300 can be reconfigured in this manner.

The device 300 is also provided with outer buttons 326, one or both of which can be depressed inwardly from a defined extended position (as depicted in the figure) to a defined depressed position (not shown) to impart a curve to the channel, or at least increase the compression of the second array of fins 356 against the conduit 324. This reconfiguration of the curvature or width of the channel/compression of the conduit may provide for additional pull resistance in some circumstances. An effective narrowing of the channel 304 by depression of one or both buttons 326 may also enable the device 300 to be used with a wider range of conduit diameters.

The buttons may be coupled to the support portion 310 and optionally provided with a deflectable inner wall portion (not shown), generally as disclosed herein, to apply inward forces along a length of the channel 304.

A securement device 400 may in some embodiments be provided with an open support portion 410. As shown in Figures 4(a) and 4(b), this facilitates rotation of the second inner portion 412b around co-moulded pins or protrusions 428, which rest in apertures 430 in the support portion 410.

Further components of a base portion, such as a securement arrangement or external attachment may be removably attached to the body in use, to allow for rotation or reconfiguration of the devices 300, 400.

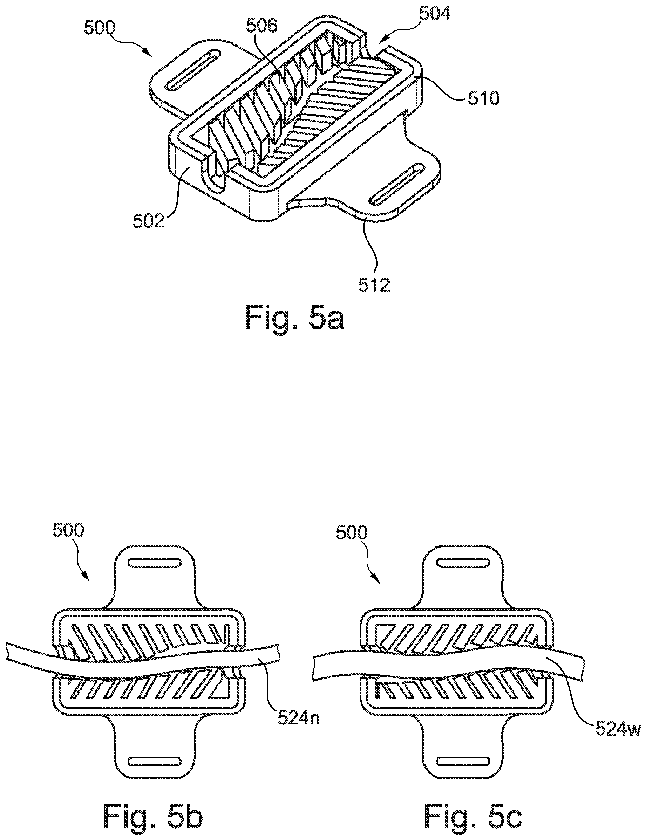

Another securement device 500 is shown in Figure 5(a). Features in common with the device 100 are provided with like reverence numerals incremented by 400.

The device 500 has a body 502, having a support portion 510. The body 502 (in particular, the inner portion 512 thereof) defines a convoluted channel 504. With the array of fins 506 in an undeformed configuration as shown in the figure, the channel 504 is convoluted and comprises two curves along its length. For certain application, in particular in use with a flexible conduit such as a IV line, a the curved channel 504 supplements the pull resistance of the device 500, because tension applied to the conduit longitudinally acts to straighten the conduit along the channel and urge the conduit against the fins on one side of a curve of the channel 504. The fins are thus further resiliently deformed and friction between the conduit and the channel is increased.

In addition, a curved or convoluted pathway may be better able to accommodate a range of conduit diameters. This is illustrated in Figures 5(b) and 5(c) which show, respectively, a narrow 524n and a wide 524w IV line secured within the channel 504. In the embodiment shown the narrow IV line 524n is a 3mm diameter line and the wide IV line 524w is a 6 mm diameter line. The curvature provides for at least some of the fins 506 to engage with the narrow conduit and provide a suitable pull resistance.

It will be understood that a securement device in accordance with the invention can be configured to accommodate different diameter flexible conduit, or a smaller or wider range of conduit diameters. For example, in some settings 10 mm or 15 mm fluid conduits may be used and a given securement device may be adapted to accommodate both diameters.

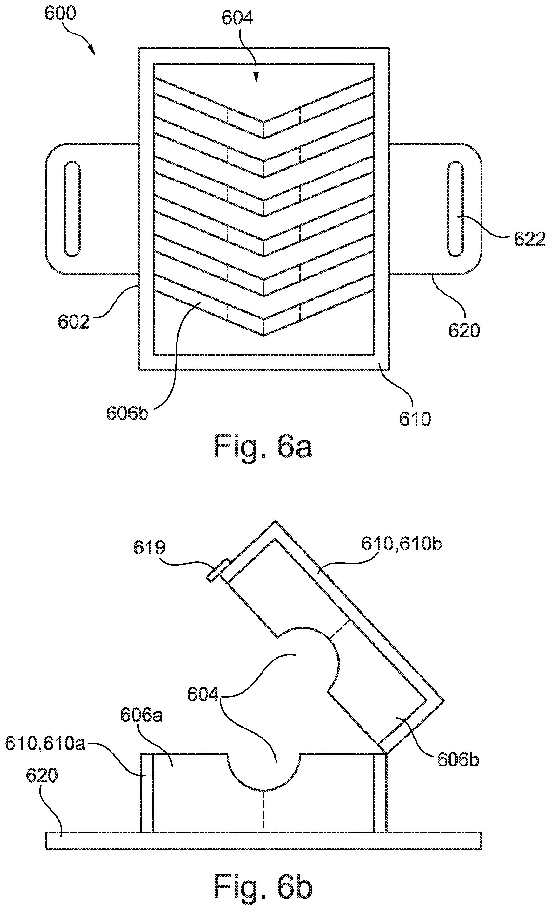

Figures 6(a) and 6(b) show plan and cross sectional end views of another conduit securement device 600. Features in common with the device 100 are provided with like reference numerals, incremented by 500.

The device 600 has a body 602 that defines a channel 604. In the embodiment shown, the channel is straight, but curved or convoluted embodiments are also contemplated. The device also includes a base 620, with slots 622 therein, which function as an external attachment as described above.

The body 602 comprises an outer support portion 610 that includes a lower part 610a and a lid part 610b, coupled via an active hinge 611. Each part 610a, 610b of the support portion 610 defines a cavity with which is mounted a lower stiffness (e.g. silicone) lower and upper inner portion 612a, 612b, respectively.

The lower inner portion 612a defines a first sub-array of fins 606a. The upper inner portion 612b defines a second sub-array of fins 606b. The first and second sub-arrays comprise fins that are oriented in the same direction as one another.

As shown in Figure 6(b) each sub-array of fins 606a, 606b includes a cut-out of semi-circular cross section from each pair of opposed fins (as viewed along the channel), whereby when the lid 610b is closed, the sub-arrays 606a, 606b come together to define an array of fins 606 extending around the channel.

In cross section, when the lid 610b is closed (and latched via latch 619) and when the fins are undeformed, the array 606 defines channel having a void pathway 605 of circular cross section. Void pathways having other cross sections are also contemplated, as disclosed herein.

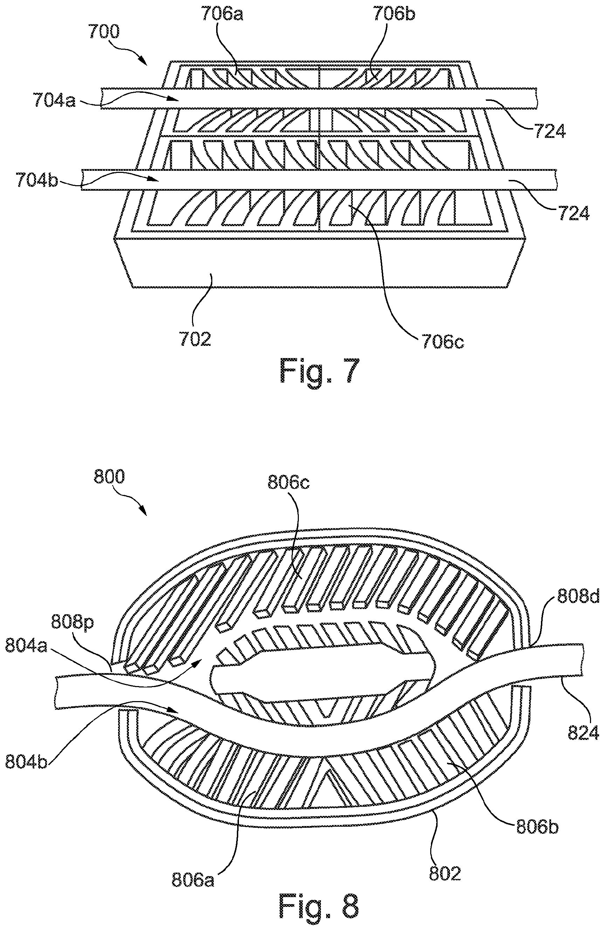

Figures 7 and 8 show devices 700, 800 having a body 702, 802 defining two channels.

The device 700 has a first channel 704a and a parallel second channel 704b. The first channel 704a has two arrays of fins 706a and 706b in opposite orientations, generally as described above for the device 300. The second channel 704b has a single array of fins 706c, generally as described above for the device 100. The device 700 allows a user to select a channel for a conduit 724 between a strong pull resistance in one direction, or pull resistance in both directions, is required. Two conduits can be used simultaneously.

In alternative embodiments, the device can alternatively or additionally or be provided with different widths of channel, channels with different stiffness fins etc., or combinations of such options.

The device 800 has a first channel 804a and a second channel 804b sharing common proximal and distal ends 808p, 808d. The channels are curved. The second channel 804b has two arrays of fins 806a and 806b in opposite orientations. The first channel 804b has a single array of fins 806c. A conduit 824 may be routed along either channel.

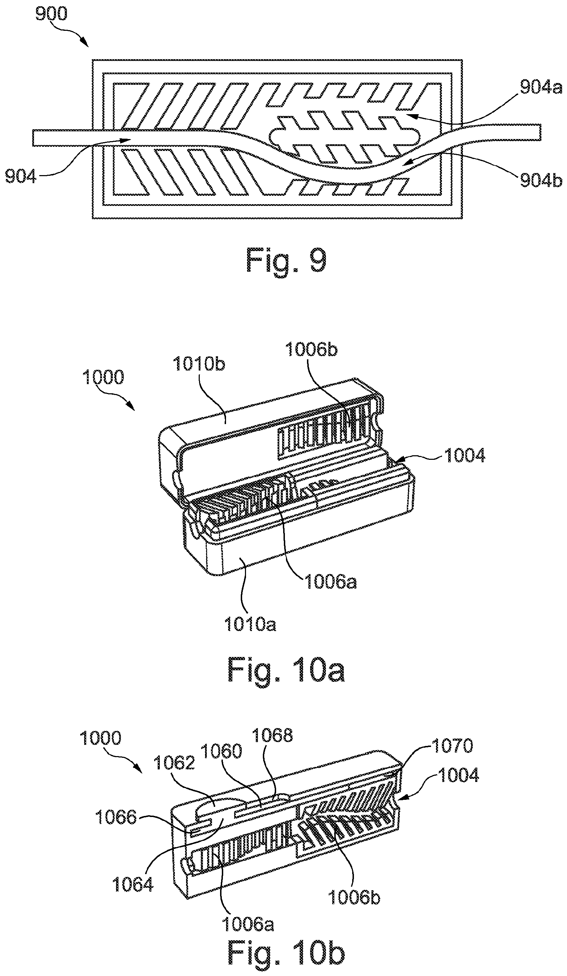

The securement device 900 of Figure 9 has a branched channel arrangement, comprising a main channel 904 that branches to a first channel 904a and a second channel 904b, sharing common proximal and distal ends. The first and second channels comprise fins arrayed in opposite directions from one another, and provide for a choice of flexible conduit routing in use.

Figure 10(a) shows a reconfigurable securement device 1000 having a convoluted channel 1004. The device 1000 has a first channel portion with a first array of fins 1006a extending from first and opposed second sides, and a second channel portion with a second array of fins 1006b extending from orthogonal third and opposed fourth sides. The device 1000 has a support portion having a lower part 1010a that defines a recess housing a first inner portion 1012a comprising the first array 1006a. A further recess houses a lower component of the second array 1006b. An upper part 1010b hinged to the lower part 1010a houses an upper component of the second array 1006b, and further functions as a retention arrangement when the upper and lower parts are closed around a conduit in use. As shown in the cross sectional view of Figure 10(b), the support portion 1010 includes a slider 1060 comprising a button 1062 that extends outwardly of the support portion, connected via a neck 1064 to a moveable wall portion 1066. The neck extends through a slot 1068, whereby the button may be operated to move the wall portion 1066. The wall potion is housed within a recess 1070 adjacent the first array 1006a defined within the support portion 1010. Adjacent the second array 1006b, the recess is defined between the second array 1006b and the support portion 1010. Movement of the wall portion 1066 adjusts the length of the second array (upper part) that is able to move within the recess, and thus provides for some adjustment of the force applied by the second array 1006b against a conduit within the channel 1004 in use.

In some applications, operation of the slider moves the second array 1006b into and out of engagement with a conduit, thereby providing a user with the option of reconfiguring the device to have a single direction, or two directions of strong pull resistance. This could be convenient for example to adjust the position of a conduit without needing to open the lid 1010b, to optimise placement of the conduit. For example, in some procedures precise advancement of a conduit might be required, but withdrawal of the conduit might be detrimental. Once such example is advancing a catheter into an artery, such as the femoral artery towards the heart to reduce bleeding in the aorta, wherein use of the device may ensure that the catheter cannot be pushed back out of the artery during the procedure (which could be life threatening). Once the conduit is adequately placed, the device could be reconfigured to provide the "two way" pull resistance.

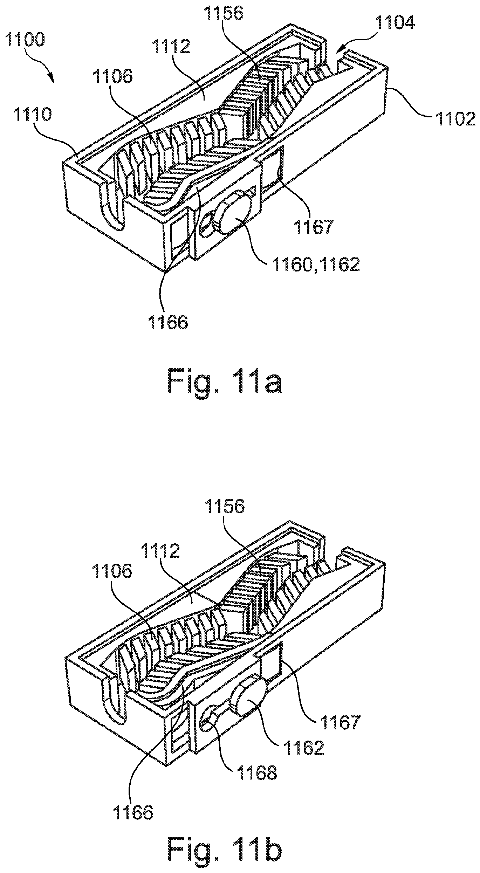

Figures 11(a) and 11(b) show another securement device 1100 with a convoluted channel 1104. The body 1102 defines first and second arrays 1106, 1156 oriented in opposite directions. The device 1100 is reconfigurable between two configurations as shown in the figures. The body comprises an outer support portion 1110 having a slider 1160, with a button 1162 connected via a neck (not visible) to a button internal to the support portion (not visible). The internal button abuts a movable wall portion 1166 that flexes from a pivot 1167. Movement of the slider within the slot 1168 causes the internal button to slide against the moveable wall portion 1166 closer (Figure 11(b)) or further (Figure 11(a)) from the pivot 1167, thereby adjusting the degree of inward flex of the wall portion 1166. The wall portion 1166 abuts the inner portion 1112 and so provides for a degree of adjustment of the width of the channel 1104 (i.e. the void pathway thereof) and, in use, the force applied between the fins 1106 and a conduit within the channel, in the region of the channel adjacent the wall portion 1166. Again, and as discussed above, without removing the conduit from the device, the device can in use provide a pull resistance in a single direction, then be reconfigured to provide additional pull resistance in the opposite direction once a conduit placement has been finalised.

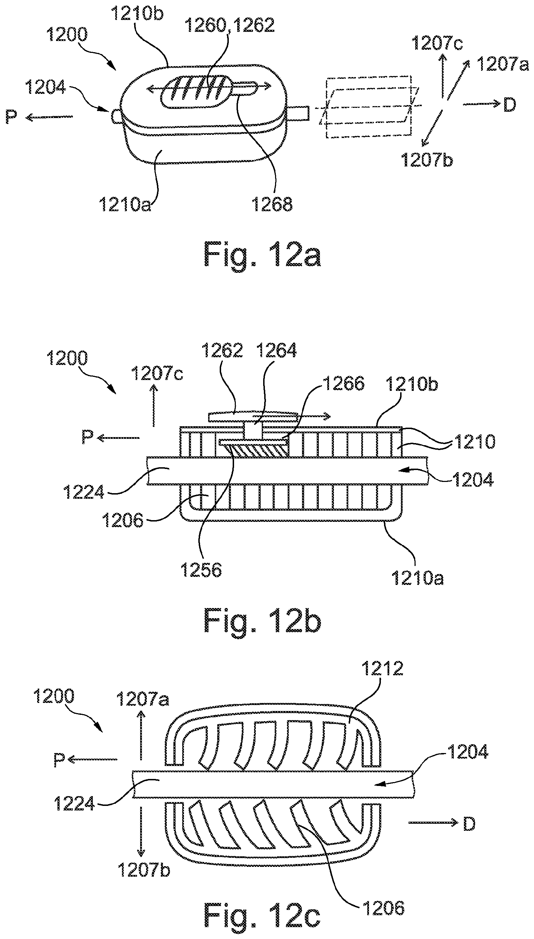

Figures 12(a)-12(c) show another example of a reconfigurable securement device 1200. The device 1200 has a straight channel 1204 having a first array of fins 1206 extending from opposed first and second sides 1207a, 1207b of the channel 1204. The fins 1206 form part of a resiliently deformable insert portion 1212a, within the cavity of the lower part 1210a of a support portion 1210. The fins of the array 1206 are oriented in a direction D.

The support portion 1210 also includes a lid 1210b. The lid 1210b includes a slider 1260 comprising a button 1262 that extends outwardly of the support portion, connected via a neck 1264 to a moveable wall portion 1266. The neck extends through a slot 1268, whereby the button may be operated to move the wall portion 1266. A second array of fins 1256 is attached to the moveable wall portion and, when the lid 1210b is closed, extends along a portion of the length of the channel from a third side 1207c thereof.

When a conduit 1224 is inserted into the channel 1204 and the lid 1210b closed over the lower portion 1210a, the fins of the first array 1206 engage with and are resiliently deformed against the conduit 1224, and provide a greater pull resistance to longitudinal forces applied between the conduit 1224 and the device 1224 in the direction D, as compared to the pull resistance to forces applied in the direction P.

When the button 1260 is in the position furthest in the direction P in the slot 1268, as shown in the figures, the fins 1256 are resiliently deformed so as to be oriented in the direction P, i.e. the opposite orientation to the orientation of the fins of the first array 1206a.

When the button 1260 is moved to the distal end of the slot (as far as possible in the direction D) the fixed ends of the fins 1206b are pulled together with the moveable wall portion such that the orientation of the fins 1206b is reversed. The compressive forces within the fins biases the button 1260 in one or other of these two positions.

The second array of fins 1256 of the device 1200 is thus reconfigurable to adjust the contribution of the fins 1256 to the overall pull resistance of the device in each direction.

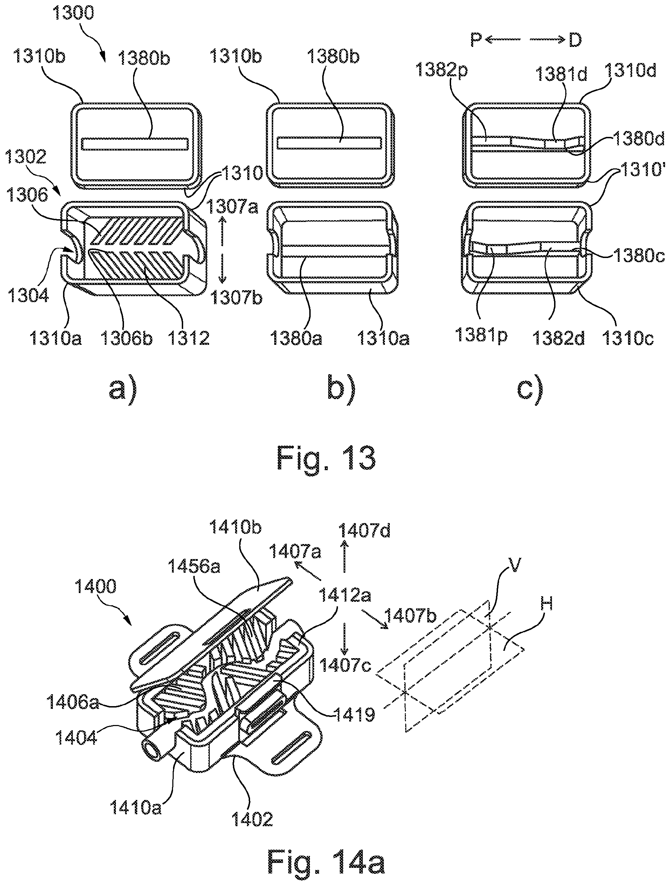

Figure 13(a) shows a conduit securement device 1300 in which the body 1302 includes a support portion 1310 having a lower portion 1310a and an upper portion 1310b in the form of a lid. An insert potion 1312 comprising an array of fins 1306 is positioned in a recess defined by the lower portion 1310a. The fins extend from first and opposite second sides 1307a, 1307b of the channel 1304, and the channel includes a void pathway between the free ends 1306b of the fins 1306.

The void pathway is also bounded to orthogonal third and fourth sides by a lower rib 1380a and an upper rib 1380b, which can be most clearly be seen in Figure 13(b), which shows the body with the insert portion 1312 removed. In the embodiment shown, the ribs are co-moulded with the support portion 1310 of the body and are rigid. In alternative embodiments, resiliently deformable ribs, or ribs with resiliently deformable portions may be provided, as disclosed herein.

The ribs 1380a, 1380b engage with the free ends 1306b of the fins 1306 where they extend between the fins, and so inhibit eversion or prolapse of the fins 1306. The ribs can also be considered to facilitate use of a more conformable, less resilient material for construction of the fins, which can be advantageous for providing a required pull resistance without risk of over-compressing and pinching or occluding a conduit, in some applications.

In the embodiments of the lower portion 1310a and the lid 1310b, the ribs 1380a, 1380b are a constant depth, such that the channel 1304 is straight, and the void pathway has a rectangular cross section.

Figure 13(c) shows an alternative support portion 1310' with alternative lower portions 1310c and upper lid portions 1310d for use with the same insert portion 1312. The support portion 1310' has undulating ribs 1380c and 1380d that undulate in the third and fourth directions in relation to the channel. The lower rib 1380c has a proximal trough 1381p and a distal peak 1382d, whereas the upper rib 1380d has a complimentary proximal peak 1382p and distal trough 1381d.

Thus, the channel 1304 is planar in the vertical plane (defined between the fins) and undulating in and out of a horizonal plane through the device 1300.

Figures 14(a)-14(c) show a securement device 1400 having a convoluted channel 1404 with multiple directions of curvature.

The device 1400 has a body 1402, with a base 1420 and slots 1420 that function as an external attachment, as discussed above. The body 1402 has a lower support portion 1410a, attached via an active hinge (not visible in Figure 14(a)) to an upper lid portion 1410b, and securable over the channel by a latch 1419.

A first insert portion 1412a moulded from a resilient material is located in a recess of the lower support portion 1410a, and comprises first and second arrays of fins 1406a and 1456a having opposite orientations, extending from first and second sides 1407a, 1407b of the channel 1404.

In a plane H parallel to the first and second directions (i.e. horizontally through the device 1400 in the orientation shown in the figures), the channel 1404 is convoluted and has three curves along its length.

The device 1400 also includes a second insert portion 1412b having a lower part 1413a and an upper part 1413b set into the lower support portion 1410a and the lid 1410b, respectively. The second insert portion 1412b defines third and fourth arrays of fins 1406b and 1456b, of opposite orientations to one another, each extending from third and opposed fourth sides 1407c, 1407d of the channel 1407c, 1407d that are orthogonal to the first and second sides.

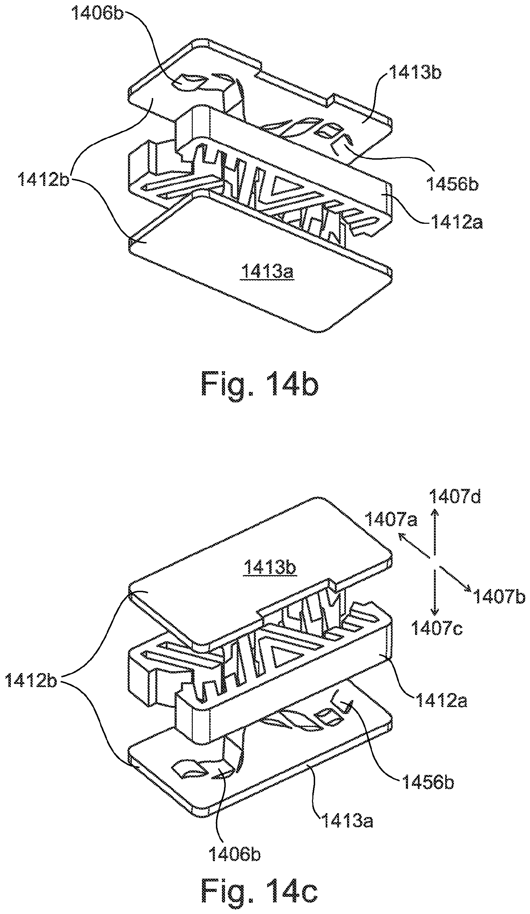

Figures 14(b) and (c) show exploded perspective views of the first and second insert portions, and illustrate that the channel 1404 includes a void pathway between the free ends of the various fins, and that the fins of the third and fourth array 1456a, 1456b extend in the direction of the plane V (vertical in the orientation shown in the figures) partially between the free ends of the first and second arrays 1406a, 1456a.

Analogously to the ribs 1380c and 1380d of the device 1300, the free ends of fins of the third and fourth array, as viewed parallel to the plane H, define complimentary undulations. Consequently, in the plane V parallel to the third and fourth directions, the channel 1404 also is convoluted and has three curves along its length.

It should be further noted that the free ends of the first and second arrays are generally longitudinally aligned with the free ends of the third and fourth arrays, such that the third and fourth arrays act to inhibit eversion or prolapse of the fins of the first and second arrays, in use. In the embodiment shown, the third and fourth arrays of fins are generally shorter than those of the first and second arrays and thus less prone to prolapse.

It will be understood that the terms vertical, horizontal, upper and lower, and any other terms relating to orientation refer to the relative orientation of various features of the devices disclosed herein, such as in relation to the orientation of devices shown in the figures. Such terms are not to be construed as limiting upon the orientation of the device or features in use.

It will be understood that the various optional features discussed above (regarding a retention arrangement, external attachment, etc) may be provided in connection with any of the examples or embodiments. Further, that the various features disclosed in relation to any of the examples or embodiments (such as but not limited to means for reconfiguring, channel shape, size, lids/closures, construction methods or materials, arrays and sub-arrays) may also be provided in further combinations with one another.

## Claims

1. A conduit securement device (300, 400, 700, 900, 1100), comprising:
a body defining a channel (304, 704a, 1104), for receiving a length of a conduit (324, 724) extending along the channel; and
a first array of resiliently deformable fins (306, 406, 706a, 1106) disposed along the channel extending from at least a first side and an opposite second side of the channel; and
a second array (356, 456, 706b, 1156) of resiliently deformable fins disposed along the channel extending from at least a first side and an opposite second side of the channel;
wherein each fin has an orientation that extends at an acute angle (α) to a longitudinal direction (L) along the channel;
wherein each of the fins of the first array has a first orientation with respect to the said longitudinal direction along the channel, and each of the fins of the second array has an opposite second orientation with respect to the said longitudinal direction along the channel; and
wherein the first array extends along a first portion of a length of the channel and the second array extends along a second portion of the length of the channel.

2. The conduit securement device (900) of claim 1, wherein the channel (904a) is curved or convoluted, along at least a part of the length of the channel, optionally wherein the channel has two, or more than two directions of curvature along its length.

3. The conduit securement device (300, 400, 1100) of any preceding claim, wherein the body comprises an inner portion (312a, 412a, 1112) and a support portion (310, 410, 1110) disposed laterally outside of the inner portion along at least the first and second sides of the channel (304, 1104), wherein the inner portion defines the channel and comprises the arrays of resiliently deformable fins (306, 406, 1106; and 356, 456, 1156);
wherein the support portion comprises a material having a first stiffness and at least the fins of the inner portion comprise a material having a lower second stiffness.

4. The conduit securement device (300, 400, 1100) of claim 3, wherein inner portion (312a, 412a, 1112) and support portion (310, 410, 1110) are removably coupled to one another; or wherein the inner portion is co-moulded within the support portion, and/or wherein the support portion comprises or defines a recess, and the inner portion is located in the recess.

5. The conduit securement device (300, 400, 1100) of claim 3 or 4, wherein at least the fins of the inner portion (312a, 412a, 1112) comprise or consist of an elastomeric material, such as a silicone;
and/or wherein at the support portion (310, 410, 1110), or at least a part thereof, is rigid or semi-rigid and is formed from a moulded or machined plastics material, such as polypropylene.

6. The conduit securement device (300) of any preceding claim, wherein the body further comprises a retention arrangement for resisting or preventing movement of a conduit (324) laterally of out the channel (304), optionally wherein the retention arrangement comprises one or more straps, which can be releasably secured across the channel, or wherein the retention arrangement comprises a lid or cover, for attaching over at least a part of the length of the channel.

7. The conduit securement device (300) of any preceding claim, wherein, when the fins (306, 356) are in an undeformed state, the channel (304) defines a void pathway between opposed fins, optionally wherein, when the fins are undeformed, in cross section through the channel the ends of the opposed fins define two, three or four sides of a square, rectangular or diamond shaped in cross section void pathway; or wherein the ends of the array of fins facing the channel may define a part of a circle, oval or polygon.

8. The conduit securement device (300, 400, 700, 900, 1100) of any preceding claim, wherein, in an undeformed state, the fins are substantially straight, between their fixed and free ends; or wherein the fins are curved or J-shaped between their fixed and free ends.

9. The conduit securement device (300, 400, 1100) of any preceding claim, wherein the securement device is reconfigurable, between at least a first configuration and a second configuration, to change an angle of at least some of the fins in relation to the channel (304, 1104), change a width of the void pathway along at least a part of a length of the channel, change a curvature of the channel, or change the orientation of at least some of the fins, or the like.

10. The conduit securement device (300, 400, 1100) of claim 9, wherein the securement device is reconfigurable by removing and replacing one or more fin components: optionally wherein each array (306, 356; 406, 456; and 1106, 1156) comprises a removably coupled fin component, whereby the device can be reconfigured by removing the fin component and recoupling the fin component with the fins thereof in an opposite orientation; or wherein the first array or the second array comprises a rotatable fin component, rotatably coupled to the body or support portion, whereby the securement device is reconfigurable by rotating said fin components to reverse the orientation of the fins thereof.

11. The conduit securement device (700, 900) of any preceding claim, comprising a first channel (704a, 904a) and a second channel (704b, 904b), optionally wherein the first and second channels may share a common first end and/or a common second end; and/or further comprising a branched channel arrangement.

12. The conduit securement device (300, 400, 700, 900, 1100) of any preceding claim, further comprising an external attachment, for attaching the securing device to a patient or structure, wherein the external attachment is coupled to the body.

13. A medical system comprising a medical conduit (324, 724) secured to a conduit securement device (300, 400, 700, 900, 1100) according to any preceding claim, wherein the conduit extends along a length of a channel (304, 704a, 904a, 1104) of the securement device.

14. A method of securing a conduit comprising:
providing a conduit securing device (300, 400, 700, 900, 1100) in accordance with any one of claims 1 to 12; and
inserting a conduit (324, 724) laterally into a channel (304, 704a, 904a, 1104) thereof.

15. The method according to claim 14, comprising operating a retention arrangement, by securing a strap over the channel (304, 704a, 904a, 1104), or closing a lid over the channel.

## Patentansprüche

1. Leitungssicherungsvorrichtung (300, 400, 700, 900, 1100), umfassend:
einen Körper, der einen Kanal (304, 704a, 1104) zum Aufnehmen einer Länge einer Leitung (324, 724) definiert, die sich entlang des Kanals erstreckt; und
eine erste Anordnung aus elastisch verformbaren Lamellen (306, 406, 706a, 1106), die entlang des Kanals angeordnet sind und sich von zumindest einer ersten Seite und einer gegenüberliegenden zweiten Seite des Kanals erstrecken; und
eine zweite Anordnung (356, 456, 706b, 1156) aus elastisch verformbaren Lamellen, die entlang des Kanals angeordnet sind und sich von zumindest einer ersten Seite und einer gegenüberliegenden zweiten Seite des Kanals erstrecken;
wobei jede Lamelle eine Ausrichtung aufweist, die sich in einem spitzen Winkel (α) zu einer Längsrichtung (L) entlang des Kanals erstreckt;
wobei jede der Lamellen der ersten Anordnung eine erste Ausrichtung in Bezug auf die Längsrichtung entlang des Kanals aufweist und jede der Lamellen der zweiten Anordnung eine entgegengesetzte zweite Ausrichtung in Bezug auf die Längsrichtung entlang des Kanals aufweist; und
wobei sich die erste Anordnung entlang eines ersten Abschnitts einer Länge des Kanals erstreckt und sich die zweite Anordnung entlang eines zweiten Abschnitts der Länge des Kanals erstreckt.

2. Leitungssicherungsvorrichtung (900) nach Anspruch 1, wobei der Kanal (904a) entlang zumindest eines Teils der Länge des Kanals gekrümmt oder gewunden ist, optional wobei der Kanal entlang seiner Länge zwei oder mehr als zwei Krümmungsrichtungen aufweist.

3. Leitungssicherungsvorrichtung (300, 400, 1100) nach einem der vorhergehenden Ansprüche, wobei der Körper einen inneren Abschnitt (312a, 412a, 1112) und einen Stützabschnitt (310, 410, 1110) umfasst, der seitlich außerhalb des inneren Abschnitts entlang zumindest der ersten und zweiten Seite des Kanals (304, 1104) angeordnet ist, wobei der innere Abschnitt den Kanal definiert und die Anordnungen aus elastisch verformbaren Lamellen (306, 406, 1106; und 356, 456, 1156) umfasst;
wobei der Stützabschnitt ein Material mit einer ersten Steifigkeit umfasst und zumindest die Lamellen des inneren Abschnitts ein Material mit einer geringeren zweiten Steifigkeit umfassen.

4. Leitungssicherungsvorrichtung (300, 400, 1100) nach Anspruch 3, wobei der innere Abschnitt (312a, 412a, 1112) und der Stützabschnitt (310, 410, 1110) lösbar miteinander gekoppelt sind; oder wobei der innere Abschnitt innerhalb des Stützabschnitts im Verbund geformt ist und/oder wobei der Stützabschnitt eine Aussparung umfasst oder definiert und sich der innere Abschnitt in der Aussparung befindet.

5. Leitungssicherungsvorrichtung (300, 400, 1100) nach Anspruch 3 oder 4, wobei zumindest die Lamellen des inneren Abschnitts (312a, 412a, 1112) ein Elastomermaterial umfassen oder daraus bestehen,
wie beispielsweise ein Silikon;
und/oder wobei der Stützabschnitt (310, 410, 1110) oder zumindest ein Teil davon starr oder halbstarr ist und aus einem geformten oder bearbeiteten Kunststoffmaterial, wie etwa Polypropylen, gebildet ist.

6. Leitungssicherungsvorrichtung (300) nach einem der vorhergehenden Ansprüche, wobei der Körper ferner eine Halteeinrichtung zum Widerstehen oder Verhindern einer Bewegung einer Leitung (324) seitlich aus dem Kanal (304) heraus umfasst, optional wobei die Halteeinrichtung ein oder mehrere Bänder umfasst, die lösbar über dem Kanal gesichert werden können, oder wobei die Halteeinrichtung einen Deckel oder eine Abdeckung zum Befestigen über zumindest einen Teil der Länge des Kanals umfasst.

7. Leitungssicherungsvorrichtung (300) nach einem der vorhergehenden Ansprüche, wobei, wenn sich die Lamelllen (306, 356) in einem unverformten Zustand befinden, der Kanal (304) einen Hohlraumpfad zwischen gegenüberliegenden Lamellen definiert, optional wobei, wenn die Lamellen unverformt sind, die Enden der gegenüberliegenden Lamellen im Querschnitt durch den Kanal zwei, drei oder vier Seiten eines im Querschnitt quadratischen, rechteckigen oder rautenförmigen Hohlraumpfads definieren; oder wobei die dem Kanal zugewandten Enden der Lamellenanordnung einen Teil eines Kreises, Ovals oder Polygons definieren können.

8. Leitungssicherungsvorrichtung (300, 400, 700, 900, 1100) nach einem der vorhergehenden Ansprüche, wobei die Lamellen in einem unverformten Zustand zwischen ihren festen und freien Enden im Wesentlichen gerade sind; oder wobei die Lamellen zwischen ihren festen und freien Enden gekrümmt oder J-förmig sind.

9. Leitungssicherungsvorrichtung (300, 400, 1100) nach einem der vorhergehenden Ansprüche, wobei die Sicherungsvorrichtung zwischen zumindest einer ersten Konfiguration und einer zweiten Konfiguration neu konfigurierbar ist, um einen Winkel von zumindest einigen der Lamellen in Bezug auf den Kanal (304, 1104) zu ändern, eine Breite des Hohlraumpfads entlang zumindest eines Teils einer Länge des Kanals zu ändern, eine Krümmung des Kanals zu ändern oder die Ausrichtung von zumindest einigen der Lamellen zu ändern oder Ähnliches.

10. Leitungssicherungsvorrichtung (300, 400, 1100) nach Anspruch 9, wobei die Sicherungsvorrichtung durch Entfernen und Ersetzen einer oder mehrerer Lamellenkomponenten neu konfigurierbar ist: optional wobei jede Anordnung (306, 356; 406, 456; und 1106, 1156) eine lösbar gekoppelte Lamellenkomponente umfasst, wodurch die Vorrichtung durch Entfernen der Lamellenkomponente und erneutes Koppeln der Lamellenkomponente mit den Lamellen davon in entgegengesetzter Ausrichtung neu konfiguriert werden kann; oder wobei die erste Anordnung oder die zweite Anordnung eine drehbare Lamellenkomponente umfasst, die drehbar mit dem Körper oder dem Stützabschnitt gekoppelt ist, wodurch die Sicherungsvorrichtung durch Drehen der Lamellenkomponenten zum Umkehren der Ausrichtung der Lamellen davon neu konfigurierbar ist.

11. Leitungssicherungsvorrichtung (700, 900) nach einem der vorhergehenden Ansprüche, umfassend einen ersten Kanal (704a, 904a) und einen zweiten Kanal (704b, 904b), optional wobei der erste und der zweite Kanal ein gemeinsames erstes Ende und/oder ein gemeinsames zweites Ende gemeinsam nutzen können; und/oder ferner umfassend eine verzweigte Kanaleinrichtung.

12. Leitungssicherungsvorrichtung (300, 400, 700, 900, 1100) nach einem der vorhergehenden Ansprüche, ferner umfassend eine externe Befestigung zum Befestigen der Sicherungsvorrichtung an einem Patienten oder einer Struktur, wobei die externe Befestigung mit dem Körper gekoppelt ist.

13. Medizinisches System, umfassend eine medizinische Leitung (324, 724), die an einer Leitungssicherungsvorrichtung (300, 400, 700, 900, 1100) gemäß einem der vorhergehenden Ansprüche gesichert ist, wobei sich die Leitung entlang einer Länge eines Kanals (304, 704a, 904a, 1104) der Sicherungsvorrichtung erstreckt.

14. Verfahren zum Sichern einer Leitung, umfassend:
Bereitstellen einer Leitungssicherungsvorrichtung (300, 400, 700, 900, 1100) in Übereinstimmung mit einem der Ansprüche 1 bis 12; und
und seitliches Einführen einer Leitung (324, 724) in einen Kanal (304, 704a, 904a, 1104) davon.

15. Verfahren gemäß Anspruch 14, umfassend das Bedienen einer Halteeinrichtung durch Sichern eines Bandes über dem Kanal (304, 704a, 904a, 1104) oder Schließen eines Deckels über dem Kanal.

## Revendications

1. Dispositif de fixation de conduit (300, 400, 700, 900, 1100), comprenant :
un corps définissant un canal (304, 704a, 1104), destiné à recevoir une longueur d'un conduit (324, 724) s'étendant le long du canal ; et
un premier ensemble d'ailettes élastiquement déformables (306, 406, 706a, 1106) disposées le long du canal s'étendant depuis au moins un premier côté et un second côté opposé du canal ; et
un second ensemble (356, 456, 706b, 1156) d'ailettes déformables de manière élastique disposées le long du canal s'étendant depuis au moins un premier côté et un second côté opposé du canal ;
dans lequel chaque ailette présente une orientation qui s'étend à un angle aigu (α) par rapport à une direction longitudinale (L) le long du canal ;
dans lequel chacune des ailettes du premier ensemble comporte une première orientation par rapport à ladite direction longitudinale le long du canal, et chacune des ailettes du second ensemble comporte une seconde orientation opposée par rapport à ladite direction longitudinale le long du canal ; et
dans lequel le premier ensemble s'étend le long d'une première partie d'une longueur du canal et le second ensemble s'étend le long d'une seconde partie de la longueur du canal.

2. Dispositif de fixation de conduit (900) de la revendication 1, dans lequel le canal (904a) est incurvé ou convoluté, sur au moins une partie de la longueur du canal, éventuellement dans lequel le canal comporte deux, ou plus de deux directions de courbure sur sa longueur.

3. Dispositif de fixation de conduit (300, 400, 1100) d'une quelconque revendication précédente, dans lequel le corps comprend une partie interne (312a, 412a, 1112) et une partie de support (310, 410, 1110) disposée latéralement à l'extérieur de la partie interne le long d'au moins les premier et deuxième côtés du canal (304, 1104), dans lequel la partie interne définit le canal et comprend les ensembles d'ailettes élastiquement déformables (306, 406, 1106 ; et 356, 456, 1156) ;
dans lequel la partie de support comprend un matériau comportant une première rigidité et au moins les ailettes de la partie interne comprennent un matériau comportant une seconde rigidité inférieure.

4. Dispositif de fixation de conduit (300, 400, 1100) de la revendication 3, dans lequel partie interne (312a, 412a, 1112) et partie de support (310, 410, 1110) sont couplées de manière amovible l'une à l'autre ; ou dans lequel la partie interne est co-moulée à l'intérieur de la partie de support, et/ou dans lequel la partie de support comprend ou définit un évidement, et la partie interne est située dans l'évidement.

5. Dispositif de fixation de conduit (300, 400, 1100) de la revendication 3 ou 4, dans lequel au moins les ailettes de la partie interne (312a, 412a, 1112) comprennent ou sont constituées d'un matériau élastomère, tel qu'un silicone ;
et/ou dans lequel la partie de support (310, 410, 1110), ou au moins une partie de celle-ci, est rigide ou semi-rigide et est formée à partir d'un matériau plastique moulé ou usiné, tel que du polypropylène.

6. Dispositif de fixation de conduit (300) d'une quelconque revendication précédente, dans lequel le corps comprend en outre un agencement de retenue destiné à résister au mouvement d'un conduit (324) latéralement hors du canal (304) ou à empêcher celui-ci, éventuellement dans lequel l'agencement de retenue comprend une ou plusieurs sangles, qui peuvent être fixées de manière amovible à travers le canal, ou dans lequel l'agencement de retenue comprend un couvercle ou un revêtement, destiné à être fixé sur au moins une partie de la longueur du canal.

7. Dispositif de fixation de conduit (300) d'une quelconque revendication précédente, dans lequel, lorsque les ailettes (306, 356) sont dans un état non déformé, le canal (304) définit un chemin vide entre des ailettes opposées, éventuellement dans lequel, lorsque les ailettes ne sont pas déformées, en section transversale à travers le canal, les extrémités des ailettes opposées définissent deux, trois ou quatre côtés d'un chemin vide de section transversale en forme de carré, de rectangle ou de losange ; ou dans lequel les extrémités de l'ensemble d'ailettes faisant face au canal peuvent définir une partie d'un cercle, d'un ovale ou d'un polygone.

8. Dispositif de fixation de conduit (300, 400, 700, 900, 1100) d'une quelconque revendication précédente, dans lequel, dans un état non déformé, les ailettes sont sensiblement droites, entre leurs extrémités fixe et libre ; ou dans lequel les ailettes sont incurvées ou en forme de J entre leurs extrémités fixe et libre.

9. Dispositif de fixation de conduit (300, 400, 1100) d'une quelconque revendication précédente, dans lequel le dispositif de fixation peut être reconfiguré, entre au moins une première configuration et une seconde configuration, pour modifier un angle d'au moins certaines des ailettes par rapport au canal (304, 1104), modifier une largeur du trajet de vide le long d'au moins une partie d'une longueur du canal, modifier une courbure du canal, ou modifier l'orientation d'au moins certaines des ailettes, ou similaire.

10. Dispositif de fixation de conduit (300, 400, 1100) de la revendication 9, dans lequel le dispositif de fixation peut être reconfiguré en retirant et en remplaçant un ou plusieurs composants d'ailettes : éventuellement dans lequel chaque ensemble (306, 356 ; 406, 456 ; et 1106, 1156) comprend un composant d'ailette couplé de manière amovible, moyennant quoi le dispositif peut être reconfiguré en retirant le composant d'ailette et en recouplant le composant d'ailette avec les ailettes de celui-ci dans une orientation opposée ; ou dans lequel le premier ensemble ou le second ensemble comprend un composant d'ailette rotatif, couplé de manière rotative au corps ou à la partie de support, moyennant quoi le dispositif de fixation peut être reconfiguré en faisant tourner lesdits composants d'ailettes pour inverser l'orientation des ailettes de ceux-ci.

11. Dispositif de fixation de conduit (700, 900) d'une quelconque revendication précédente, comprenant un premier canal (704a, 904a) et un second canal (704b, 904b), éventuellement dans lequel les premier et second canaux peuvent partager une première extrémité commune et/ou une seconde extrémité commune ; et/ou comprenant en outre un agencement de canal ramifié.

12. Dispositif de fixation de conduit (300, 400, 700, 900, 1100) d'une quelconque revendication précédente, comprenant en outre une fixation externe, destinée à fixer le dispositif de fixation à un patient ou à une structure, dans lequel la fixation externe est couplée au corps.

13. Système médical comprenant un conduit médical (324, 724) fixé à un dispositif de fixation de conduit (300, 400, 700, 900, 1100) selon une quelconque revendication précédente, dans lequel le conduit s'étend le long d'une longueur d'un canal (304, 704a, 904a, 1104) du dispositif de fixation.

14. Procédé de fixation d'un conduit comprenant :
la fourniture d'un dispositif de fixation de conduit (300, 400, 700, 900, 1100) selon l'une quelconque des revendications 1 à 12 ; et
l'insertion latérale d'un conduit (324, 724) dans un canal (304, 704a, 904a, 1104) de celui-ci.

15. Procédé de la revendication 14, comprenant l'utilisation d'un agencement de retenue, en fixant une sangle sur le canal (304, 704a, 904a, 1104), ou en fermant un couvercle sur le canal.
